# EUROPÄISCHE PATENTSCHRIFT

(11) **EP 1 013 640 B1**
(45) Veröffentlichungstag und Bekanntmachung des Hinweises auf die Patenterteilung: **10.09.2003**
(21) Anmeldenummer: 99125660.3
(22) Anmeldetag: 22.12.1999
(51) Int. Cl.: C07C 277/08

(54) **Synthese von Guanidinderivaten ausgehend von O-alkylisoharnstoffsalzen unter Anwendung von Impfkristallen**
Synthesis of guanidines from O-alkylisoureas using a crystal seed
Procédé pour la préparation de guanidines à partir de O-alkylisourées en utilisant des cristaux de germination

(30) Priorität: 23.12.1998 DE 19860048
(43) Veröffentlichungstag der Anmeldung: 28.06.2000
(73) Patentinhaber: BASF AKTIENGESELLSCHAFT, 67056 Ludwigshafen (DE)
(72) Erfinder: Kessel, Knut, 68161 Mannheim (DE); Kluge, Michael, 67071 Ludwigshafen (DE); Greindl, Thomas, 67098 Bad Dürkheim (DE); Bogenstätter, Thomas, 67098 Bad Dürkheim (DE); Scherr, Günter, 67065 Ludwigshafen (DE); Rauls, Matthias, 67117 Limburgerhof (DE); van Gelder, Richard, 67061 Ludwigshafen (DE)
(74) Vertreter: Kinzebach, Werner, Dr.

(56) Entgegenhaltungen:
- EP-A- 0 915 083
- DE-A- 1 518 852
- PATENT ABSTRACTS OF JAPAN vol. 004, no. 030 (C-002), 15. März 1980 (1980-03-15) & JP 55 004349 A (IWAI TOMIRO), 12. Januar 1980 (1980-01-12)
- SCHWETLICK ET AL.: "ORGANIKUM" 1977 , VEB DEUTSCHER VERLAG DER WISSENSCHAFTEN XP002133400 * seite 46: "Impfkristall" bei "übersättigten Lösungen" * * Seite 44 - Seite 46 *
- R.B. FEARING ET AL.: "Some strongly basic derivatives of (+) and (-)-1-Hydroxy-2-aminobutane" JOURNAL OF THE AMERICAN CHEMICAL SOCIETY, Bd. 76, 5. September 1954 (1954-09-05), Seiten 4382-4385, XP002133399

## Beschreibung

Die vorliegende Erfindung betrifft ein Verfahren zur Herstellung von Guanidinderivaten der Formel I 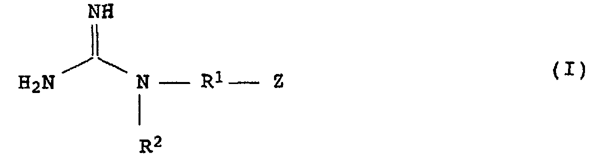 worin
- R¹: für C₁-C₈-Alkylen einen zweiwertigen cycloaliphatischen Rest mit 5 bis 10 Kohlenstoffatomen
- R²: für H, C₁-C₈-Alkyl steht, oder
- R¹: und R² gemeinsam mit dem N-Atom, an das sie gebunden sind, für einen 5- oder 6-gliedrigen mit Z substituierten Ring stehen,
- Z: für COOR³, SO₂OR³ oder PO(OR³)(OR⁴),
- R³: jeweils unabhängig für H, ein Alkalimetall oder ein Äquivalent eines Erdalkalimetalls, und
- R⁴: jeweils unabhängig für H, ein Alkalimetall, ein Äquivalent eines Erdalkalimetalls oder C₁-C₆-Alkyl steht,
durch Umsetzung eines O-Alkylisoharnstoffsalzes der Formel II, 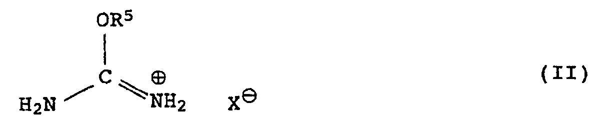 worin
- R⁵: für C₁-C₈-Alkyl und X^{⊖} für ein Äquivalent eines Anions steht, mit einem Amin der Formel III, 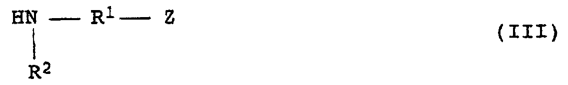
worin
- R¹, R² und Z: die angegebene Bedeutung besitzen.

Die Erfindung betrifft insbesondere ein verfahren zur Herstellung von Kreatin aus einem O-Alkylisoharnstoffsalz und Sarkosinat.

Guanidinverbindungen der Formel I sind in der Natur weit verbreitet. Wichtige Vertreter dieser Substanzklasse sind beispielsweise Verbindungen wie Arginin und Kreatin. Kreatin kommt im Muskelgewebe der Wirbeltiere insbesondere als Kreatinphosphat vor und spielt eine wichtige Rolle als Energieträger der Zelle. Kreatin findet zunehmend Verwendung als Nahrungsergänzungsmittel zur Stärkung der körperlichen Leistungsfähigkeit. Außerdem findet Kreatin Verwendung zur Behandlung von Muskelfunktionsstörungen, die durch erhöhte Kreatinausscheidung im Urin gekennzeichnet sind.

Die EP-A-0754679 beschreibt ein Verfahren zur Herstellung von Kreatin oder Kreatin-Monohydrat aus Cyanamid und Natrium- oder Kaliumsarkosinat in Wasser oder einem Gemisch aus Wasser und einem organischem Lösungsmittel. Ein Nachteil dieses Verfahrens ist die Verwendung wässriger Lösungen von reinem Cyanamid. Diese Lösungen sind sehr teuer und aufgrund der Instabilität des Cyanamids im Allgemeinen nicht breit verfügbar.

Aus der DE-A-38 12 208 ist ein Verfahren zur Herstellung von 4-Guanidinomethylcyclohexancarbonsäure bekannt, bei dem man 4-Aminomethylcyclohexancarbonsäure mit einem O-Alkylisoharnstoff-Derivat in einem polaren protischen Lösemittel umsetzt.

Die JP-A-53/077364 beschreibt ein Verfahren zur Herstellung von Kreatin, bei dem nach und nach ein O-Alkylisoharnstoff-Salz zu einer wässrigen Sarkosinlösung gegeben wird, die ständig im pH-Bereich von 10 bis 12 gehalten wird. Dieses Verfahren zeigt jedoch bestimmte Nachteile, die es für eine großtechnische Herstellung von Kreatin ungeeignet erscheinen lassen. So wurde festgestellt, dass die Reaktionsmischung mit zunehmendem Umsatz zunehmend viskoser und schwieriger zu rühren wird. Das erhaltene Kreatin-Monohydrat weist dabei eine Reinheit von lediglich 97% auf. Außerdem zeigt das erhaltene Produkt eine sehr breite Teilchengrößenverteilung mit einer sehr niedrigen mittleren Teilchengröße. Dies führt zu einer unzureichenden Filtrierbarkeit des Produkts. Der nach dem Trocknen erhaltene hohe Feingutanteil ist aufgrund des Staubens des Produkts beim Verpacken bzw. Umfüllen unerwünscht. Zwar kann das Produkt, z.B. durch Umkristallisation, gereinigt und die Teilchengröße eingestellt werden, aber der zusätzliche Verfahrensschritt ist wegen der geringen Löslichkeit von Kreatin extrem aufwendig und wegen der geringen Temperaturabhängigkeit der Löslichkeit von Kreatin mit hohen Verlusten an Produkt verbunden.

Der vorliegenden Erfindung liegt daher die Aufgabe zugrunde, das eingangs genannte Verfahren so weiterzubilden, dass auf einfache Weise kristalline Guanidinderivate der Formel I in hoher Reinheit, hoher Ausbeute und mit enger Teilchengrößenverteilung erhalten werden; zudem soll die Teilchengröße so eingestellt werden können, dass gut filtrierbare Kristallsuspensionen erhalten werden.

Erfindungsgemäß wird diese Aufgabe dadurch gelöst, dass die Umsetzung von O-Alkylisoharnstoffsalz der Formel II mit dem Amin der Formel III in Gegenwart von Impfkristallen des Guanidinderivats der Formel I erfolgt. Es sind über die gesamte Dauer der Umsetzung Impfkristalle vorhanden. Vorzugsweise erfolgt die Umsetzung in Gegenwart von 1 bis 40 Gew.-%, insbesondere 2 bis 35 Gew.-%, besonders bevorzugt 5 bis 20 Gew.-%, bezogen auf das Reaktionsgemisch, Impfkristallen. Die angegebenen Werte gelten für die diskontinuierliche Verfahrensführung bzw. die kontinuierliche Verfahrensführung nach Erreichen des stationären Zustandes. Zum Anfahren sind bei letzterer auch geringere Mengen an Impfkristallen, z.B. etwa 0,1 Gew.-%, ausreichend.

Die Anmelderin hat in umfangreichen Versuchen festgestellt, dass bei dem Verfahren nach der JP-A-53/077364 die Konzentration an Kreatin im Reaktionsgemisch zeitweise die Sättigungskonzentration um das 5-fache übersteigt, da das System zur Bildung einer metastabilen übersättigten Phase neigt und die Kristallisation kinetisch verzögert ist. Dies hat zur Folge, dass bei sehr hohen Übersättigungen eine spontane Kristallkeimbildung einsetzt. Dabei werden viele Kristallkeime gleichzeitig gebildet, so dass ein Produkt mit einem hohen Anteil kleiner Teilchen erhalten wird. Bei der spontanen Kristallisation aus übersättigter Lösung werden außerdem Verunreinigungen aus dem Reaktionsmedium mitgerissen.

Das erfindungsgemäße Verfahren umgeht diese Nachteile, indem das Inkontaktbringen und die Umsetzung von O-Alkylisoharnstoff der Formel II und Amin der Formel III in Gegenwart vorgelegter Kristalle von Guanidinderivat der Formel I erfolgen, die als Impfkristalle für die Abscheidung von Produkt dienen. Auf diese Weise kann die übersättigung des Reaktionsgemisches an Guanidinderivat der Formel I unterhalb eines Werts gehalten werden, bei dem spontane Kristallkeimbildung einsetzt. Außerdem wird auf Grund des durch die Anwesenheit der Impfkristalle bedingten geringeren Anteils an in Lösung befindlichem Guanidinderivat der Formel I dessen Hydrolyse durch das in der Regel stark basische Reaktionsmedium zurückgedrängt

Das erfindungsgemäße Verfahren bedient sich des Einsatzes eines O-Alkylisoharnstoffsalzes der Formel II. Eine bevorzugte Möglichkeit zur Herstellung von O-Alkylisoharnstoffsalzen besteht in der Umsetzung von Harnstoff mit Alkylierungsmitteln, wie Dialkylsulfaten. Zweckmäßigerweise wird das O-Alkylisoharnstoffsalz nach einem Verfahren erhalten, bei dem Harnstoff und ein Alkylgruppen-übertragendes Reagenz in einem kontinuierlich betriebenen Reaktor bei einer Temperatur von 40-200°C umgesetzt werden. Dabei kann mit Vorteil ein Teilstrom des in öliger Form anfallenden O-Alkylisoharnstoffsalzes zur Lösung des eingesetzten Harnstoffs zurückgeführt werden. Das zurückgeführte O-Alkylisoharnstoffsalz wirkt außerdem als Katalysator der Alkylierungsreaktion und als Verdünnungsmittel, das den Temperaturanstieg bei der exothermen Reaktion begrenzt und eine thermische Zersetzung des gebildeten thermolabilen O-Alkylisoharnstoffsalzes verhindert. Als kontinuierlich betriebener Reaktor für die Umsetzung von Harnstoff und Alkylgruppen-übertragendem Reagenz, z.B. Dimethylsulfat, ist ein Rohrreaktor besonders geeignet.

Alternativ können O-Alkylisoharnstoffe durch Umsetzung von wasserfreiem Cyanamid mit Alkoholen in Gegenwart von Säuren erhalten werden.

R⁵ in der Formel II steht für verzweigtes oder unverzweigtes C₁-C₈-Alkyl, insbesondere
Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 2,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl. Bevorzugt sind C₁-C₄-Alkyl, wie Methyl, Ethyl, Propyl, iso-Propyl, Butyl, wobei Methyl besonders bevorzugt ist.

In der Formel II steht X für ein Äquivalent eines Anions, wie ein Halogenid, z.B. Cl⁻, Br⁻, I⁻, oder ein C₁-C₈-Alkylsulfat, z.B. Methylsulfat oder Ethylsulfat, Sulfat, Hydrogensulfat. O-Methylisoharnstoff-methylsulfat ist am stärksten bevorzugt.

Als weiterer Ausgangsstoff kommt ein Amin der Formel III zum Einsatz. R¹ steht für einen verzweigten oder unverzweigten C₁-C₈-Alkylenrest, vorzugsweise
Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1,1-Dimethylpropylen, 2,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen; oder einen zweiwertigen cycloaliphatischen Rest mit 5 bis 10 Kohlenstoffatomen, wie 1,2-, 1,3-, 1,4-Cyclohexylen, 1,2- oder 1,3-Cyclopentylen oder Reste folgender Struktur: 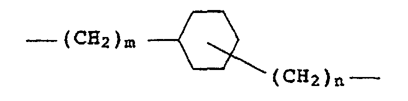 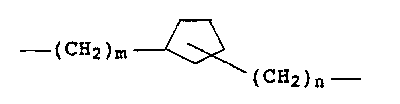 worin n und m die Werte 0, 1 oder 2 einnehmen können, mit der Maßgabe, dass n und/oder m von 0 verschieden sind. In den cycloaliphatischen Resten kann sowohl cis- als auch trans-Konfigurationen vorliegen.

Bevorzugt sind C₁-C₄-Alkylen, wie Methylen und Ethylen.

Der Rest R¹ kann mit einem oder mehreren, z.B. 1 bis 3, Substituenten, ausgewählt unter einer gegebenenfalls geschützten Amino-, Hydroxy- oder Cyanogruppe, substituiert sein.

R² steht für H oder C₁-C₈-Alkyl, vorzugsweise für H, Methyl oder Ethyl.

Alternativ können R¹ und R² gemeinsam mit dem N-Atom, an das sie gebunden sind, für einen 5- oder 6-gliedrigen mit Z substituierten Ring, z.B. einen Piperidin- oder Pyrrolidinring, stehen.

Z steht für COOR³, SO₂OR³, oder PO(OR³)(OR⁴), worin R³ jeweils unabhängig für H, ein Alkalimetall, wie Natrium oder Kalium, oder ein Äquivalent eines Erdalkalimetalls, wie Calcium, und R⁴ jeweils unabhängig für H, ein Alkalimetall, ein Äquivalent eines Erdalkalimetalls oder C₁-C₆-Alkyl steht.

Bei dem Amin der Formel III handelt es sich vorzugsweise um eine Aminocarbonsäure, Aminosulfonsäure oder Aminophosphonsäure oder ein Salz davon. Besonders bevorzugt sind Sarkosin, insbesondere in Form von Natrium- oder Kaliumsarkosinat, sowie Glycin, Taurin oder 4-Aminomethylcyclohexancarbonsäure.

Erfindungsgemäß werden das O-Alkylisoharnstoffsalz der Formel II und das Amin der Formel III in Gegenwart von Impfkristallen des Guanidinderivats der Formel I miteinander in Kontakt gebracht. So kann z.B. das O-Alkylisoharnstoffsalz, vorzugsweise in Form einer wässrigen Lösung, mit den Impfkristallen vermischt werden und die erhaltene Suspension mit dem Amin der Formel III, vorzugsweise in Form einer wässrigen Lösung, umgesetzt werden. Andererseits kann das Amin der Formel III mit den Impfkristallen vermischt werden und anschließend mit dem O-Alkylisoharnstoffsalz gemischt werden. In einer weiteren Alternative können die Impfkristalle als Pulver oder in Form einer Suspension vorgelegt werden, und das O-Alkylisoharnstoffsalz der Formel II und das Amin der Formel III werden zu den vorgelegten Impfkristallen gegeben. Soweit durch den Kontext nicht anders vorgegeben, ist die Richtung oder Reihenfolge der Zugabe einer Lösung oder einer Suspension zu einer anderen Lösung oder Suspension beliebig.

Das O-Alkylisoharnstoffsalz der Formel II und das Amin der Formel III werden vorzugsweise in einem molaren Verhältnis von 2:1 bis 1:2, insbesondere von 1,5:1 bis 0,9:1, eingesetzt.

Die erfindungsgemäße Umsetzung findet in der Regel in einem wässrigen Medium, wie z.B. Wasser selbst oder einem Gemisch von Wasser mit wassermischbaren organischen Lösungsmitteln, wie Alkoholen, z.B. Methanol oder Ethanol; Aceton oder THF, statt. Das Reaktionsmedium enthält in der Regel den bei der Umsetzung freiwerdenden Alkohol R⁵OH; gegebenenfalls kann man zusätzliche Mengen des Alkohols R⁵OH zugeben.

Die erfindungsgemäße Umsetzung findet vorzugsweise bei einem pH-Wert von 6 bis 14, insbesondere 8,5 bis 12,5 besonders bevorzugt 9,5 bis 12 statt. Zur Einhaltung eines pH-Wertes im angegebenen Bereich ist es in der Regel erforderlich, in das Reaktionsmedium eine Base einzuführen. Geeignete Basen sind z.B. Alkalimetallhydroxide, wie NaOH, insbesondere als etwa 50%ige Lösung; KOH, insbesondere als etwa 50%ige Lösung; und LiOH, besonders als etwa 10%ige Lösung, sowie Erdalkalimetallhydroxide, wie Ca(OH)₂. Die einzusetzende Base wird mit Vorteil so ausgewählt, dass das Salz des mit der Base eingeführten Kations mit dem Anion X^{⊖} aus dem O-Alkylisoharnstoffsalz der Formel II eine möglichst hohe Löslichkeit im Reaktionsmedium zeigt. So ist z.B. in Fällen, in denen X^{⊖} für ein Alkylsulfat, Hydrogensulfat oder ein Äquivalent von Sulfat steht, die Verwendung von Alkalimetallhydroxiden gegenüber der Verwendung von Erdalkalimetallhydroxiden bevorzugt.

Mitunter können Gemische von Alkalimetallhydroxiden eingesetzt werden, deren gemischte Salze mit dem X^{⊖}-Anion eine höhere Löslichkeit zeigen als die reinen Salze.

Die Einführung der Base geschieht vorzugsweise bedarfsgesteuert. Hierzu wird mittels einer kontinuierlich arbeitenden pH-Wert-Messung der aktuelle pH-Wert des Reaktionsmediums gemessen, mit einem pH-Vorgabewert verglichen und die zur pH-Wert-Korrektur notwendige Basenmenge dazugegeben. Der gemessene pH-Wert kann z.B. in Form eines elektronischen Signals bereitgestellt werden, der von einem elektronischen Datenprozessor mit einem Vorgabewert verglichen wird, der automatisch die erforderliche Basenmenge berechnet und eine Basendosierungsanlage steuert.

Das erfindungsgemäße Verfahren erfolgt vorzugsweise bei einer Temperatur von -20 bis 100°C, insbesondere 0 bis 80°C, besonders bevorzugt von 5 bis 35°C. Die Umsetzung zwischen dem O-Alkylisoharnstoffsalz der Formel II und dem Amin der Formel III verläuft exotherm. Zur Einhaltung der angegebenen Temperatur ist es daher in der Regel erforderlich, das Reaktionsmedium zu kühlen. Dies erfolgt zweckmäßigerweise über geeignete Wärmetauschvorrichtungen. Alternativ kann kontinuierlich eine Teilmenge des Reaktionsmediums über eine Temperierstrecke umgepumpt werden. Die Reaktorverweilzeiten beim erfindungsgemäßen Verfahren betragen im Allgemeinen 0,5 bis 120, bevorzugt 5 bis 72, ganz besonders bevorzugt 12 bis 48 Stunden.

Das erfindungsgemäße Verfahren kann bei Überdruck, Normaldruck oder unter Vakuum, z.B. bei einem absoluten Druck bis hinunter zu 10 mbar, durchgeführt werden. Im Allgemeinen ist das Arbeiten unter Normaldruck bevorzugt. Das Arbeiten unter Vakuum kann Vorteile bringen, indem der als Reaktionskoppelprodukt gebildete Alkohol R⁵OH bzw. das gegebenenfalls durch Nebenreaktionen gebildete Ammoniak kontinuierlich aus dem Reaktionsgemisch entfernt werden können, wodurch ein unerwünschter Mehrverbrauch an O-Alkylisoharnstoffsalz vermieden werden kann. Außerdem kann durch das Arbeiten unter Vakuum ein Siedekühlungseffekt erzielt werden.

Das erfindungsgemäße Verfahren kann absatzweise, semi-kontinuierlich oder kontinuierlich durchgeführt werden, wird aber vorzugsweise kontinuierlich durchgeführt. Für die kontinuierliche Durchführung des erfindungsgemäßen Verfahrens sind gängige Reaktoren geeignet, wie insbesondere ein kontinuierlicher Rührkesselreaktor oder eine Rührkesselkaskade. Zweckmäßigerweise wird für eine gute Durchmischung der Reaktanden im Reaktionsraum gesorgt.

Die kontinuierliche Verfahrensführung weist den Vorteil auf, dass im Reaktionsmedium im Wesentlichen konstante Konzentrationen der Reaktanden und eine im Wesentlichen konstante Menge an im Reaktionsmedium gelöstem und ungelöstem Guanidinderivat der Formel I vorliegt. Auf diese Weise kann ein im Wesentlichen konstanter, steuerbarer Grad an Übersättigung und damit eine kontrollierte Geschwindigkeit der Kristallisation des Guanidinderivats der Formel I eingehalten werden. Durch die Steuerung der Kristallisationsgeschwindigkeit des Guanidinderivats der Formel I können Produkte hoher Reinheit mit weitgehend einheitlicher Teilchengrößenverteilung sowie mittleren Teilchengrößen von mehr als z.B. 100 µm erhalten werden. Hierdurch kann der Filterwiderstand der Suspension z.B. auf Werte von unter 10x10¹² mPas·m⁻² abgesenkt werden und es werden praktikable Filtrationszeiten erreicht. In der Regel weisen die erhaltenen Kristalle eine hinreichend hohe Reinheit auf, so dass sie nach einfachem Waschen ohne weitere Reinigungsstufen z.B. als Nahrungsergänzungsmittel verwendet werden können.

In einer besonders bevorzugten Ausführungsform erfolgt die Umsetzung in einem Reaktionsraum, in den kontinuierlich O-Alkylisoharnstoffsalz der Formel II und Amin der Formel III eingeführt werden und aus dem kontinuierlich Kristalle des Guanidinderivats der Formel I, vorzugsweise in Form einer Kristallsuspension, entnommen werden. Zweckmäßigerweise werden das O-Alkylisoharnstoffsalz der Formel II und/oder das Amin der Formel III in Form wässriger Lösungen eingeführt. In dem Reaktionsraum müssen stets Kristalle des Guanidinderivats der Formel I vorliegen, die als Impfkristalle für das bei der Umsetzung des neu zugeführten O-Alkylisoharnstoffsalzes und Amins gebildete Produkt dienen. Dies kann z.B. dadurch erreicht werden, dass die Entnahme der Suspension von Kristallen des Guanidinderivats der Formel I so gesteuert wird, dass ein Teil der Kristalle im Reaktionsraum zurückbleibt, der als Impfkristallsaat für die weitere Kristallisation dient.

Für den Erhalt eines Produktes mit vorherbestimmbarer und einheitlicher Größenverteilung ist es bevorzugt, in den Reaktionsraum kontinuierlich Impfkristalle des Guanidinderivats der Formel I, vorzugsweise in Form einer Suspension, einzuführen. Die eingeführten Impfkristalle können dabei eine von den entnommenen Kristallen des Guanidinderivats der Formel I unterschiedliche mittlere Kristallgröße und/oder Kristallgrößenverteilung aufweisen.

In einer besonders bevorzugten Ausführungsform dieses Aspekts der Erfindung werden die dem Reaktionsraum zugeführten Impfkristalle dadurch erhalten, dass die aus dem Reaktionsraum entnommenen Kristalle des Guanidinderivats der Formel I einer Größenklassierung unterworfen werden, wobei ein Feingutanteil und ein Grobgutanteil gewonnen wird und der Feingutanteil als Impfkristalle in den Reaktionsraum zurückgeführt und der Grobgutanteil als Produkt aus dem Verfahren ausgeleitet wird. Der Feingutanteil, der als Impfkristalle geeignet ist, weist z.B. einen Teilchengrößenbereich von 1 bis <100 µm auf. Der Grobgutanteil weist z.B. einen Teilchengrößenbereich von 100 µm bis 500 µm auf.

Die Größenklassierung der Kristalle kann sowohl in suspendiertem als auch in trockenem Zustand erfolgen. Zur Klassierung im trokkenen Zustand werden die Kristalle zuvor von der Mutterlauge getrennt und getrocknet. Der z.B. durch Siebung gewonnene Feingutanteil kann zur Rückführung in den Reaktionsraum in einem wässrigen Medium suspendiert werden. Geeignete Vorrichtungen zur Größenklassierung sind z.B. Hydrocyclone und Naßsiebe für die Klassierung suspendierter Kristalle und Cyclone, Siebe oder Sichter für die Klassierung getrockneter Kristalle.

Das eingesetzte O-Alkylisoharnstoffsalz liegt üblicherweise in fester Form oder in Form eines hochviskosen Öls vor. Es hat sich gezeigt, dass es beim Eintragen des O-Alkylisoharnstoffsalzes in die alkalische Reaktionslösung in Substanz an der Phasengrenzfläche zwischen O-Alkylisoharnstoffsalz und Reaktionsmedium zu einer lokalen Überhitzung kommt. Die lokale Überhitzung begünstigt Nebenreaktionen gegenüber der Bildung des gewünschten Guanidinderivats. Werden als O-Alkylisoharnstoffsalz Alkylsulfate eingesetzt, so begünstigt die lokale Überhitzung außerdem die Hydrolyse des Alkylsulfatanions zu Sulfat, was einen unerwünschten Mehrverbrauch an Base bedingt. Die Alkali- oder Erdalkalisulfate sind außerdem schlechter löslich als die entsprechenden Alkylsulfate. Außerdem ist das feste oder hochviskose O-Alkylisoharnstoffsalz auch mit hochwirksamen Mischern nur schwer in die Reaktionslösung einzumischen. Die eingetragene Mischenergie wird dann bisweilen so groß, dass größere Kristalle des Guanidinderivats der Formel I infolge der Einwirkung von Scherkräften zerstört werden. Es ist daher bevorzugt, das eingesetzte O-Alkylisoharnstoffsalz in Form einer wässrigen Lösung mit einem Gehalt von in der Regel 85 bis 15, insbesondere 70 bis 30 Gew.-% einzusetzen.

Die wässrige Lösung weist dabei in der Regel eine Viskosität von 500 bis 1 mPas, insbesondere 100 bis 1 mPas und besonders bevorzugt 10 bis 1 mPas auf. Zum Ansetzen dieser Lösung des O-Alkylisoharnstoffsalzes kann ein wässriges Medium, wie Wasser selbst, verwendet werden. Gemäß einem bevorzugten Aspekt der Erfindung wird zur Zubereitung der wässrigen Lösung des O-Alkylisoharnstoffsalzes ein Waschmedium verwendet, das bei der Wäsche der erhaltenen Kristalle des Guanidinderivats der Formel I mit einem wässrigen Medium anfällt. Auf diese Weise wird zum einen der Wasserverbrauch des Gesamtverfahrens minimiert, zum anderen wird der Anteil an Guanidinderivat der Formel I, der sich beim Waschen des erhaltenen,Produkts im Waschmedium löst, in Form der Lösung des O-Alkylisoharnstoffsalzes in den Reaktionsraum zurückgeführt und bleibt im Gesamtsystem erhalten.

Das Amin der Formel III kann mit Vorteil in Form einer wäßrigen Lösung, z.B. mit einer Konzentration von 85 bis 15, insbesondere 70 bis 30 Gew.-%, eingesetzt werden. Eine geeignete Lösung ist z.B. eine handelsübliche wässrige Lösung von Natriumsarkosinat mit einem Gehalt von 40% und einer Reinheit von 85-90%.

Das Einbringen des O-Alkylisoharnstoffsalzes der Formel II und/oder des Amins der Formel III oder der wässrigen Lösungen davon sowie gegebenenfalls erforderlicher Base in das Reaktionsmedium kann zweckmäßigerweise so erfolgen, dass aus dem Reaktionsraum eine Teilmenge des Reaktionsmediums entnommen wird, mit dem O-Alkylisoharnstoffsalz und/oder dem Amin und/oder der Base vermischt wird und in den Reaktionsraum zurückgeführt wird. Die Entnahme, das Vermischen und Zurückführen erfolgen vorzugsweise kontinuierlich, z.B. durch Umpumpen von Reaktionsmedium über eine Dosierungs- und Mischstrecke, in der O-Alkylisoharnstoffsalz und/oder Amin oder wässrige Lösungen davon und/oder Base eingespeist werden. Auf diese Weise lassen sich die gewünschten Konzentrationen zweckmäßig einstellen. Lokale Konzentrationsspitzen und pH-Schwankungen können vermieden werden.

Aus der als Produktstrom entnommenen Suspension von Kristallen des Guanidinderivats der Formel I wird das Produkt nach üblichen Verfahren, z.B. durch Filtration oder Zentrifugation, abgetrennt. Geeignete Vorrichtungen, wie Drucknutschen, Vakuumbandfilter, Drehtrommelfilter und Zentrifugen sind dem Fachmann bekannt. Durch Ausleiten der zurückbleibenden Mutterlauge kann eine Anhäufung von Verunreinigungen, die in dem eingesetzten O-Alkylisoharnstoffsalz der Formel II und/oder dem Amin der Formel III enthalten sind, im System verhindert werden. Die aus dem System entnommene Mutterlauge dient somit insbesondere als Ausleitstrom für die beim erfindungsgemäßen Verfahren entstehenden Koppelprodukte in Form des Alkohols R⁵OH und dem Salz aus dem Kation der verwendeten Base mit dem Anion X^{⊖}.

Die von der Mutterlauge abgetrennten Kristalle können, z.B. mit kaltem oder warmem Wasser, gewaschen werden. Hierzu können die Kristalle mit dem Waschmedium aufgeschlämmt und anschließend abgetrennt werden. Das Waschmedium kann, wie oben erörtert, zum Ansetzen der Lösung des O-Alkylisoharnstoffsalzes verwendet und so in das Verfahren zurückgeführt werden.

Die von der Mutterlauge abgetrennten und gegebenenfalls gewaschenen Kristalle des Guanidinderivats der Formel I können dann nach üblichen Verfahren getrocknet werden. Hierzu sind z.B. eine pneumatische Förderung, Stromtrockner oder Wirbelbetten geeignet. Zweckmäßigerweise werden die Kristalle auf einen Gehalt an nicht im Kristall gebundener Feuchtigkeit von 5 bis 0 Gew.-%, vorzugsweise 2,5 bis 0 Gew.-%, getrocknet.

Im Einzelfall kann es vorteilhaft sein, den nach der Abtrennung von der Mutterlauge erhaltenen feuchten Kristallkuchen von Guanidinderivat der Formel I mit bereits getrocknetem Material zu vermischen und das Gemisch der weiteren Trocknung zu unterziehen, um auf diese Weise ein Verbacken beim Trocknen zu verhindern. Bei kontinuierlicher Verfahrensführung kann dies durch die Rückführung eines Teils des getrockneten Materials erreicht werden.

Je nach Temperatur und Umgebungsfeuchtigkeit ist entweder die wasserfreie Form oder ein Hydrat des Guanidinderivats der Formel I die thermodynamisch stabilste Form; gegebenenfalls werden zusätzliche Hydrate mit abweichenden Zusammensetzungen als metastabile Strukturen erhalten. Im Falle des Kreatins ist das Monohydrat die übliche Handelsform; bei der Trocknung muß gegebenenfalls nach dem Fachmann bekannten Verfahren dafür gesorgt werden, dass keine unerwünschte Übertrocknung eintritt.

Die Erfindung wird durch die folgenden Beispiele näher veranschaulicht.

### Beispiel 1 - Diskontinuierliche Kreatinsynthese in Gegenwart vorgelegter Kreatinkristalle

In einem doppelwandigen Glasreaktor wurde eine Suspension von 150 g Kreatin-Monohydrat in 150 ml kreatingesättigtem Wasser bei 15°C vorgelegt. Innerhalb von 4 Stunden wurden 1,02 kg (3,3 Mol) einer 60%igen technischen OMI-(O-Methylisoharnstoff)-Methylsulfatschmelze zudosiert, die mit 500 ml kreatingesättigtem Wasser bei 15 °C zu einer wässrigen Lösung der Viskosität 3 mPas vorverdünnt worden war. Unter Rühren wurde der pH-Wert ständig bei 11,0 gehalten, indem bedarfsgesteuert eine 15°C warme, 50%ige wässrige Lösung von NaOH (etwa 0,24 kg) eingepumpt wurde. Synchron zur Dosierung des Guanidierungsreagenzes wurden 0,825 kg (3Mol) einer 40%igen wässrigen Lösung von Sarkosin-Natriumsalz von 15°C zugegeben; die Innentemperatur wurde mittels eines Thermostaten unterhalb 20°C gehalten. Die Suspension ließ sich jederzeit problemlos rühren. Man rührte über Nacht insgesamt 20 Stunden bei 15°C weiter und drückte die Suspension anschließend mit einem Überdruck von 1,5 bar über eine gesinterte Metalldrahtgewebe-Verbundplatte ab; der Filterwiderstand in der Drucknutsche betrug 3x10¹²mPasm⁻². Der Reaktor wurde mit 200 ml Trinkwasser ausgespült und der Filterkuchen hiermit überschichtet; mit 0,5 bar Überdruck wurde das an den Kristallen haftende Wasser weitgehend abgeblasen. Der Rückstand wurde mit je 325 ml Trinkwasser zweimal aufgeschlämmt, jeweils 30 Minuten intensiv gerührt und auf etwa 10% Restfeuchte trockengesaugt. Abschließend wurde durch den Filterkuchen über Nacht wassergesättigte Luft bei Raumtemperatur gesaugt, wobei Kreatin in Form seines Monohydrates zurückblieb.

Nach Abzug der Impfkristalle bleiben 299 g (2 Mol) H₂N-C(=NH)-NMe-CH₂-COOHxH₂O zurück, was einer Ausbeute von 67% bezüglich Sarkosin-Natrium und 62% bezüglich OMI-Methylsulfat entspricht. Der per HPLC und Titration feststellbare Gehalt an Wertprodukt war größer als 99,5% und die Sulfatasche lag unter 0,1%; Verbackungen des Produkts mit einer mittleren Teilchengröße (DP₅₀) von etwa 150 µm traten während des Trocknens nicht auf.

Durch Siebanalyse einer Probe wurde folgende Teilchengrößenverteilung ermittelt:

| | |
|---|---|
| > 400 µm | 0,1 g |
| > 200 µm | 11,4 g |
| > 160 µm | 11,0 g |
| > 125 µm | 8,8 g |
| > 90 µm | 7,9 g |
| > 50 µm | 9,2 g |
| < 50 µm | 1,0 g |

### Beispiel 2 - Kontinuierliche Kreatinsynthese in Gegenwart vorgelegter Kreatinkristalle

In einem doppelwandigen Glasreaktor wurde eine Suspension von 1 g Kreatin-Monohydrat in 150 ml kreatingesättigtem Wasser bei 15°C vorgelegt. Dann wurden kontinuierlich die beiden folgenden Eduktströme (i) und (ii) synchron eindosiert: (i) 1,15 ml/min einer wässrigen Lösung der Viskosität 3 mPas, die durch Mischen von 730 ml einer 60%igen technischen OMI-Methylsulfatschmelzemit 650 ml kreatingesättigtem Wasser bei 15°C hergestellt worden war. Dies entspricht innerhalb einer mittleren Verweilzeit von 20 Stunden 3,3 Mol Edukt. 0,56 ml/min einer 15°C warmen 40%igen wässrigen Lösung von Sarkosin-Natriumsalz, was innerhalb einer mittleren Verweilzeit von 20 Stunden 3,0 Mol Edukt entspricht. Es wurde energisch gerührt und der pH-Wert ständig bei 11,0 gehalten, indem bedarfsgesteuert eine 15°C warme, 50%ige wässrige Lösung von NaOH (etwa 0,14 ml/min) eingepumpt wurde. Die Innentemperatur des Reaktors wurde mit einem Thermostaten bei 15°C gehalten. Sobald der Kolbeninhalt ein Volumen von 2100 ml erreichte, wurden über ein Ventil jeweils 100 ml der Produktsuspension abgelassen. Nach 100 h (entsprechend dem 5-fachen der mittleren Verweilzeit) ist davon auszugehen, dass im Reaktor ein stationärer Zustand erreicht ist. Daher wurde die im Reaktor verbliebene Suspension von 2100 ml Volumen mit einem Überdruck von 1,5 bar über eine gesinterte Metalldrahtgewebe-Verbundplatte abgedrückt; der Filterwiderstand in der Drucknutsche betrug 3x10¹²mPasm⁻². Der Reaktor wurde mit 200 ml Trinkwasser ausgespült und der Filterkuchen hiermit überschichtet; mit 0,5 bar Überdruck wurde das an den Kristallen haftende Wasser weitgehend abgeblasen. Der Rückstand wurde mit je 325 ml Trinkwasser zweimal aufgeschlämmt und jeweils 30 Minuten intensiv gerührt und auf etwa 10% Restfeuchte trockengesaugt. Abschließend wurde durch den Filterkuchen über Nacht wassergesättigte Luft bei Raumtemperatur gesaugt, wobei Kreatin in Form seines Monohydrates zurückblieb.

Es bleiben 246 g (1,65 Mol) H₂N-C(=NH)-NMe-CH₂-COOHxH₂O zurück, was einer Ausbeute von 55% bezüglich Sarkosin-Natrium und 50% bezüglich OMI-Methylsulfat entspricht. Der per HPLC und Titration feststellbare Gehalt an Wertprodukt war größer als 99,5% und die Sulfatasche lag unter 0,1%; Verbackungen des Produkts mit der mittleren Teilchengröße 115 µm treten während des Trocknens nicht auf.

### Beispiel 3 - Kontinuierliche Kreatinsynthese in Gegenwart vorgelegter Kreatinkristalle in einer Reaktorkaskade

In einem doppelwandigen 2 l-Glasreaktor mit Propellerrührer und Stromstörern werden 1 g Kreatin-Monohydrat sowie 300 ml kreatingesättigtes Wasser bei einer Temperatur von 15°C vorgelegt. Über ein Bodenventil wird laufend Suspension mit einer Förderrate von etwa 15 l/h über einen etwa 50 ml fassenden, eine Dosier- und Mischstrecke aufweisenden Umpumpkreislauf gepumpt, der mit einem Kühlmantel versehen ist. Innerhalb von 20 h werden in die Dosierstrecke in Fließrichtung 1,3 ml/min einer Lösung zudosiert, die aus 1530 g (4,95 mol) OMI-Methylsulfat (60%) und 490 ml kreatingesättigtem Wasser einer Temperatur von 5°C vorgemischt worden war. Bei der intensiven Vermischung sinkt der pH-Wert in der Mischstrecke um etwa 2 bis 3 pH-Stufen ab. Synchron werden in die Dosierstrecke stromabwärts zur Zudosierungsstelle des OMI-Methylsulfats 1,4 ml/min einer Raumtemperatur aufweisenden Lösung zudosiert, die aus 1240 g (4,5 mol) Sarkosin-Na-Salz (40% in Wasser; pH = 14), 200 ml Natronlauge (50%) und 490 ml kreatingesättigtem Wasser vorgemischt worden war. Bei der intensiven Vermischung steigt der pH-Wert in der Mischstrecke um etwa 1 bis 2 pH-Stufen an.

Im Glasreaktor wurde durch bedarfsgeregelte Zudosierung von 50%iger NaOH über eine Mikrodosierpumpe ein pH-Wert von 11,0 eingehalten. Die Temperatur des Reaktors wird mit einem Thermostaten auf 15°C begrenzt; die Rührerdrehzahl beträgt etwa 300 U/min. Die Kühlung des Umpumpkreislaufs wird so eingestellt, dass die umgepumpte Suspension am Ende der Dosierstrecke mit einer Temperatur von 15°C austritt.

Sobald das Flüssigkeitsvolumen im Reaktor 1050 ml erreicht, wird vor dem Eingang in die Dosierstrecke über ein pneumatisch gesteuertes Ventil Produktsuspension aus dem Umpumpkreislauf in einen doppelwandigen 2 l-Glasreaktor mit Propellerrührer abgelassen, in dem 250 ml kreatingesättigtes Wasser einer Temperatur von 15°C vorgelegt wurden. Auch in diesem Reaktor wird der pH-Wert durch bedarfsgeregelte Dosierung von 50%iger NaOH bei 11 gehalten. Sobald das Flüssigkeitsvolumen im zweiten Reaktor 1050 ml erreicht, wird über ein pneumatisch gesteuertes Bodenventil Produktsuspension in einen darunter befindlichen doppelwandigen 2-1 Glasreaktor mit Propellerrührer abgelassen, in dem 100 ml kreatingesättigtes Wasser einer Temperatur von 15°C vorgelegt wurden. Beim Erreichen eines Volumens von 1050 ml wird die Produktsuspension über einen Überlauf abgelassen.

Die Gesamtverweilzeit über die drei Reaktoren beträgt bei dieser Anordnung 20 h. Die obigen Dosierungen werden über weitere neun Verweilzeiten (180 h) fortgesetzt. Pro Verweilzeit fallen 3150 ml freifließende Produktsuspension mit einem Feststoffgehalt von etwa 15% an, die bei 25°C kontinuierlich über eine Glassinternutsche abgezogen werden.

Sofort nach beendeter Dosierung wird der Inhalt des dritten Reaktors über eine Drucknutsche mit Sintermetallboden abgedrückt. Man spült den Reaktor mit 100 ml Trinkwasser einer Temperatur von 20°C aus und überschichtet damit das Nutschgut. Anschließend wird 10 min lang mit Luft auf eine Restfeuchte von 20% trocken geblasen. Der Rückstand wird dann in der Drucknutsche zweimal mit je einer Portion von 160 ml Trinkwasser einer Temperatur von 20°C aufgeschlämmt, 30 min lang intensiv gerührt und trocken gesaugt. Über das erhaltene saugfeuchte Rohprodukt wird bei Raumtemperatur über Nacht umgebungsfeuchte Luft gesaugt. Es bleiben 156,5 g reinweiße Kristalle von Kreatin-Monohydrat zurück, entsprechend einer Ausbeute von 70%, bezogen auf Sarkosin-Na-Salz.

Die Ausbeute kann durch Variation der Verfahrensparameter, wie insbesondere der Verweilzeit, weiter optimiert werden.

### Vergleichsbeispiel

In einem doppelwandigen Glasreaktor wurden 0,3 kg (1 Mol) einer 37%igen technischen wässrigen Lösung von Natriumsarkosinat vorgelegt. Es wurde energisch gerührt und der pH-Wert ständig bei 11,0 gehalten, indem bedarfsgesteuert eine 37%ige wässrige Lösung von NaOH zugegeben wurde. Dann wurden 0,465 kg (1,5 Mol) 60%iges O-Methylisoharnstoffmethylsulfat (Viskosität 3000 mPas) beginnend bei 15°C so schnell (etwa innerhalb 4 Stunden) zugegeben, dass die makroskopisch meßbare Innentemperatur 20°C nicht überstieg. Als Ergebnis der schlechten Mischbarkeit erkennt man erhebliche Schlierenbildung (O-Methylisoharnstoffmethylsulfat weist bei Verdünnung 1:1 mit Wasser einen pH-Wert unter 1 auf), und mit zunehmender Produktbildung ließ sich der Inhalt des Ansatzes zunehmend schwerer rühren. Nach etwa 1 h setzte die Kristallisation von Kreatin ein. Die per HPLC feststellbare Menge Kreatin in Lösung überstieg dabei die Sättigungskonzentration zeitweise um das Fünffache. Man rührte über Nacht bei Raumtemperatur weiter und drückte die Suspension mit einem Überdruck von 1,5 bar über eine gesinterte Metalldrahtgewebe-Verbundplatte ab; der Filterwiderstand betrug 8x10¹⁵ mPasm⁻². Durch Nachwaschen mit 0,12 l Wasser und 0,1 l Methanol konnte das teilweise mitgefallene NaOSO₂OMe und das durch Hydrolyse gebildete Na₂SO₄x10H₂O nur unvollständig entfernt werden. Bei Raumtemperatur und normaler Luftfeuchte trocknet der Rückstand zu rohem Kreatinmonohydrat, welches 0,1 kg wog (65% Ausbeute bzgl. Natriumsarkosinat, 43% bzgl. OMI) und eine Sulfatasche von 3% aufwies. Das Produkt verbackte beim Trocknen zu harten Aggregaten, die - bei sehr breiter Verteilung - beobachtete mittlere Teilchengröße der Kristallite betrug 30 µm, was beim weiteren Arbeiten mit dem Produkt zu immenser Staubentwicklung führte.

## Patentansprüche

1. Verfahren zur Herstellung von Guanidinderivaten der Formel I, 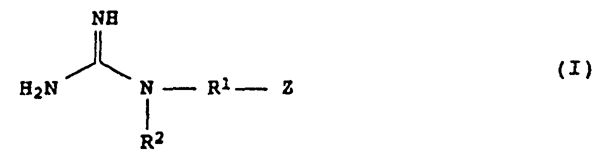 worin
R¹ für C₁-C₈-Alkylen oder einen zweiwertigen cyclo-aliphatischen Rest mit 5 bis 10 Kohlenstoffatomen steht, wobei der Rest R¹ mit einem oder mehreren Substituenten, ausgewählt unter einer gegebenenfalls geschützten Amino-, Hydroxy- oder Cyanogruppe. substituiert sein kann.
R² für H, C₁-C₈-Alkyl steht, oder
R¹ und R² gemeinsam mit dem N-Atom, an das sie gebunden sind, für einen 5- oder 6-gliedrigen mit Z substituierten Ring stehen,
Z für COOR³, SO₂OR³ oder PO(OR³)(OR⁴),
R³ jeweils unabhängig für H, ein Alkalimetall oder ein Äquivalent eines Erdalkalimetalls, und
R⁴ jeweils unabhängig für H, ein Alkalimetall, ein Äquivalent eines Erdalkalimetalls oder C₁-C₆-Alkyl steht,
durch Umsetzung eines O-Alkylisoharnstoffsalzes der Formel II, 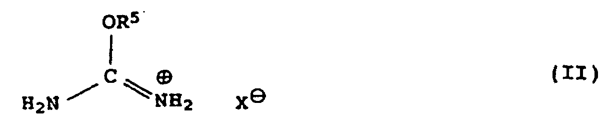 worin
R⁵ für C₁-C₈-Alkyl und X^{⊖} für ein Äquivalent eines Anions steht, mit einem Amin der Formel III, 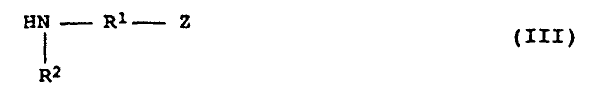
worin R¹, R² und Z die angegebene Bedeutung besitzen,
**dadurch gekennzeichnet, dass** die Umsetzung in Gegenwart von Impfkristallen des Guanidinderivats der Formel I erfolgt.

2. Verfahren nach Anspruch 1, **dadurch gekennzeichnet, dass** die Umsetzung in einem Reaktionsraum erfolgt, in den kontinuierlich O-Alkylisoharnstoffsalz der Formel II und Amin der Formel III eingeführt werden und aus dem kontinuierlich Kristalle des Guanidinderivats der Formel I entnommen werden.

3. Verfahren nach Anspruch 2, **dadurch gekennzeichnet, dass** in den Reaktionsraum kontinuierlich Impfkristalle des Guanidinderivats der Formel I eingeführt werden.

4. Verfahren nach Anspruch 3, **dadurch gekennzeichnet, dass** die entnommenen Kristalle einer Größenklassierung unterworfen werden, wobei ein Feingutanteil und ein Grobgutanteil gewonnen wird und der Feingutanteil als Impfkristalle in den Reaktionsraum zurückgeführt und der Grobgutanteil als Produkt aus dem Verfahren ausgeleitet wird.

5. Verfahren nach einem der vorhergehenden Ansprüche, **dadurch gekennzeichnet, dass** das eingesetzte O-Alkylisoharnstoffsalz in Form einer wässrigen Lösung mit einer Viskosität von 1 bis 500 mPa·s eingesetzt wird.

6. Verfahren nach Anspruch 5, **dadurch gekennzeichnet, dass** die erhaltenen Kristalle des Guanidinderivats der Formel I mit einem wässrigen Medium gewaschen werden und das Waschmedium ganz oder teilweise zur Zubereitung der wässrigen Lösung des O-Alkylisoharnstoffsalzes verwendet wird.

7. Verfahren nach einem der vorhergehenden Ansprüche, **dadurch gekennzeichnet, dass** die Umsetzung bei einem pH-Wert von 6 bis 14 erfolgt.

8. Verfahren nach einem der vorhergehenden Ansprüche, **dadurch gekennzeichnet, dass** die Umsetzung bei einer Temperatur von -20 bis 100°C erfolgt.

9. Verfahren nach einem der vorhergehenden Ansprüche, **dadurch gekennzeichnet, dass** die Umsetzung in Gegenwart von 1 bis 40 Gew.-% Impfkristallen, bezogen auf das Reaktionsgemisch, erfolgt.

10. Verfahren nach einem der vorhergehenden Ansprüche zur Herstellung von Kreatin, **dadurch gekennzeichnet, dass** es sich bei dem Amin der Formel III um Natrium- oder Kaliumsarkosinat handelt.

## Claims

1. A process for preparing guanidine derivatives of the formula I 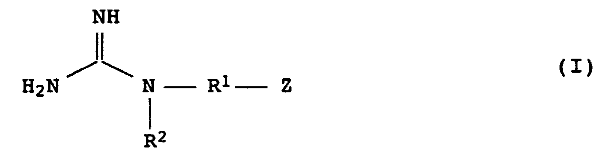 in which
R¹ is C₁-C₈-alkylene or a divalent cycloaliphatic radical with 5 to 10 carbon atoms, where the radical R¹ may be substituted by one or more substituents selected from an optionally protected amino, hydroxyl or cyano group,
R² is H, C₁-C₈-alkyl, or
R¹ and R² represent, together with the N atom to which they are bonded, a 5- or 6-membered Z-substituted ring,
Z is COOR³, SO₂OR³ or PO(OR³)(OR⁴),
R³ is in each case independently H, an alkali metal or an equivalent of an alkaline earth metal and
R⁴ is in each case independently H, an alkali metal, an equivalent of an alkaline earth metal or C₁-C₆-alkyl,
by reacting an O-alkylisourea salt of the formula II 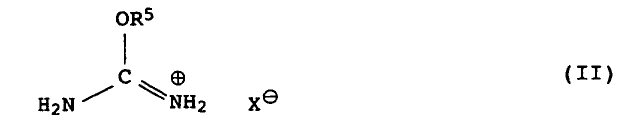 in which R⁵ is C₁-C₈-alkyl and X^{⊖} is an equivalent of an anion, with an amine of the formula III 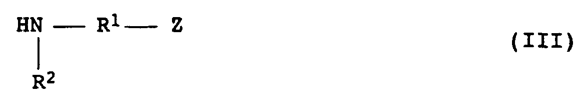 in which R¹, R² and Z have the stated meanings,
wherein the reaction takes place in the presence of seed crystals of the guanidine derivative of the formula I.

2. A process as claimed in claim 1, wherein the reaction takes place in a reaction chamber into which O-alkylisourea salt of the formula II and a amine of the formula III are continuously introduced and from which crystals of the guanidine derivative of the formula I are continuously removed.

3. A process as claimed in claim 2, wherein seed crystals of the guanidine derivative of the formula I are continuously introduced into the reaction chamber.

4. A process as claimed in claim 3, wherein the removed crystals are subjected to a size classification to result in a fine material fraction and a coarse material fraction, and the fine material fraction is returned as seed crystals to the reaction chamber, and the coarse material fraction is discharged from the process as product.

5. A process as claimed in any of the preceding claims, wherein the O-alkylisourea salt is employed in the form of an aqueous solution with a viscosity of from 1 to 500 mPa·s.

6. A process as claimed in claim 5, wherein the resulting crystals of the guanidine derivative of the formula I are washed with an aqueous medium, and the washing medium is wholly or partly used to prepare the aqueous solution of the O-alkylisourea salt.

7. A process as claimed in any of the preceding claims, wherein the reaction takes place at a pH of from 6 to 14.

8. A process as claimed in any of the preceding claims, wherein the reaction takes place at a temperature of from -20 to 100°C.

9. A process as claimed in any of the preceding claims, wherein the reaction takes place in the presence of from 1 to 40% by weight of the seed crystals based on the reaction mixture.

10. A process as claimed in any of the preceding claims for preparing creatine, wherein the amine of the formula III is sodium or potassium sarcosinate.

## Revendications

1. Procédé pour la préparation de dérivés de guanidine de formule I, 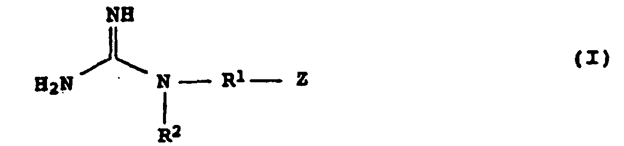 où
R¹ désigne un alkylène en C₁-C₈ ou un reste cycloaliphatique bivalent ayant 5 à 10 atomes de carbone, tandis que le reste R¹ peut être substitué par un ou plusieurs substituants, choisis parmi un groupe amino, eventuellement protégé, un groupe hydroxy ou un group cyano,
R² désigne H, un alkyle en C₁-C₈, ou
R¹ et R², conjointement avec l'atome N auquel ils sont liés, représentent un cycle à 5 ou 6 chaînons substitué par Z,
Z désigne COOR³, SO₂OR³ ou PO(OR³)(OR⁴),
R³ désigne, à chaque fois indépendamment, H, un métal alcalin ou un équivalent d'un métal alcalino-terreux, et
R⁴ désigne, à chaque fois indépendamment, H, un métal alcalin, un équivalent d'un métal alcalino-terreux ou un alkyle en C₁-C₆,
par réaction d'un sel de O-alkylisourée de formule II, 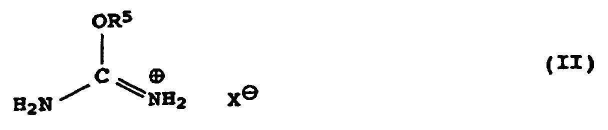 où R⁵ représente un alkyle en C₁-C₈ et X⁻ désigne un équivalent d'un anion, avec une amine de formule III, 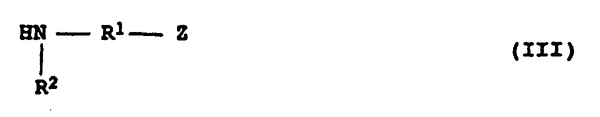 où R¹, R² et Z possèdent la signification indiquée,
**caractérisé par le fait que** la réaction est effectuée en présence de germes du dérivé de guanidine de formule I.

2. Procédé selon la revendication 1, **caractérisé par le fait que** la réaction s'effectue dans un espace réactionnel dans lequel le sel de O-alkylisourée de formule II et l'amine de formule III sont introduits en continu et d'où on soutire en continu des cristaux du dérivé de guanidine de formule I.

3. Procédé selon la revendication 2, **caractérisé par le fait qu'**on introduit en continu dans l'espace de réaction des germes du dérivé de guanidine de formule I.

4. Procédé selon la revendication 3, **caractérisé par le fait qu'**on soumet les cristaux prélevés à un tri granulométrique, tandis qu'on recueille une partie de matières fines et une partie de matières grossières, et on recycle la partie des matières fines sous forme de germes dans l'espace de réaction et la partie de matières grossières est extraite du procédé en tant que produit.

5. Procédé selon l'une des revendications précédentes, **caractérisé par le fait que** le sel de O-alkylisourée introduit est mis en oeuvre sous la forme d'une solution aqueuse ayant une viscosité de 1 à 500 mPa.s.

6. Procédé selon la revendication 5, **caractérisé par le fait que** les cristaux de dérivé de guanidine de formule I obtenus sont lavés avec un milieu aqueux et le milieu de lavage est utilisé partiellement ou en totalité pour la préparation de la solution aqueuse du sel de O-alkylisourée.

7. Procédé selon l'une des revendications précédentes, **caractérisé par le fait que** la réaction s'effectue à un pH de 6 à 14.

8. Procédé selon l'une des revendications précédentes, **caractérisé par le fait que** la réaction s'effectue à une température de -20 à 100°C.

9. Procédé selon l'une des revendications précédentes, **caractérisé par le fait que** la réaction est conduite en présence de 1 à 40 % en poids de germes, par rapport au mélange réactionnel.

10. Procédé selon l'une des revendications précédentes pour la préparation de créatine, **caractérisé par le fait qu'**il s'agit pour l'aminé de formule III de sarcosinate de sodium ou de potassium.
